# EUROPEAN PATENT APPLICATION

(11) **EP 4 506 646 A2**
(43) Date of publication of application: **12.02.2025**
(21) Application number: 24190016.6
(22) Date of filing: 22.07.2024
(51) Int. Cl.: F27D 19/00, C21D 11/00

(54) **ELECTRONIC DEVICE AND METHOD FOR CALCULATING OPTIMAL TEMPERATURE OF FURNACE**

(30) Priority: 25.07.2023 KR 20230096479
(71) Applicant: Ineeji Co., Ltd., Seongnam-si, Gyeonggi-do 13558 (KR)
(72) Inventor: KIM, Kwon Ho, 16707 Suwon-si (KR); KIM, Min Seok, 05501 Seoul (KR); Legesse, Melka Dawit, 06115 Seoul (KR); LEE, Jae Hyuk, 13562 Seongnam-si (KR); YEO, Ji Su, 46541 Busan (KR); YOO, Bo Seon, 06623 Seoul (KR)
(74) Representative: Nederlandsch Octrooibureau

(57) **Abstract**

Provided is a method of calculating an optimal temperature of a furnace, the method including: acquiring a target property value of a material input by an operator; acquiring a plurality of appropriate candidate temperatures of the furnace from first input data using an optimal temperature model of which training has been completed; acquiring predictive property values of the material from second input data using a property prediction model of which training has been completed; comparing the target property value with the predictive property values calculated by the property prediction model of which training has been completed and sampling the plurality of appropriate candidate temperatures to acquire one or more sampled appropriate candidate temperatures; acquiring heat information of a case of operating the furnace at the sampled appropriate candidate temperatures, comparing the heat information, and filtering the sampled appropriate candidate temperatures to acquire one or more appropriate temperatures; performing predetermined computation on the one or more appropriate temperatures to acquire an optimal temperature; and transmitting a request to set the acquired optimal temperature to a set temperature value of the furnace.

## Description

### CROSS-REFERENCE TO RELATED APPLICATION

This application claims priority to and the benefit of Korean Patent Application No.2023-0096479, filed on July 25, 2023, the disclosure of which is incorporated herein by reference in its entirety.

### BACKGROUND

### 1. Field of the Invention

The present disclosure relates to a technology for optimizing operation of a furnace. More particularly, the present disclosure relates to a technology for calculating an optimal temperature for operating a furnace using artificial intelligence (AI).

### 2. Discussion of Related Art

With the development of artificial intelligence (AI) technology, AI technology is being utilized in a variety of industrial fields. In particular, research has lately focused on optimizing the operation of production processes for steel products using AI models.

To produce steel products of desired quality, the properties of steel sheets should be changed through proper heat treatment in a furnace. However, the properties of steel sheets do not change linearly with heat treatment and show nonlinear relationships that change rapidly for heat treatment above an appropriate temperature. Also, the effect of the heat treatment temperature on the property changes varies depending on various factors such as the composition of the steel sheet, the thickness of the steel sheet, or the operating environment. Accordingly, it is difficult to precisely perform heat treatment that causes property changes of the steel sheets. Further, since heat treatment relies on the operator's know-how, different temperatures and operational conditions are set by different operators even when the same steel product is produced, which makes it harder to ensure quality stability. To solve this problem, technologies have been developed to optimize steel processing, but the existing steel processing optimization technologies are only used for stable control of the steel process, and the optimal operating temperature of a furnace is not calculated.

Therefore, it is necessary to develop a method of calculating the optimal temperature of a furnace for increasing thermal efficiency while ensuring quality stability of steel products and a device for performing the method.

### SUMMARY OF THE INVENTION

The present disclosure is directed to providing a method of calculating an optimal temperature of a furnace to reduce heat usage of the furnace while achieving aimed quality, and an electronic device for performing the method.

Objects of the present disclosure are not limited to that described above, and other objects which have not been described will be clearly understood by those skilled in the technical field to which the present disclosure pertains from this specification and the accompanying drawings.

According to an aspect of the present disclosure, there is provided a method of calculating an optimal temperature of a furnace, the method including: acquiring a target property value of a material input by an operator; acquiring a plurality of appropriate candidate temperatures of the furnace from first input data, which includes at least one of a thickness of the material to be heated in the furnace, a steel grade of the material, an operating speed of the furnace, and the target property value as a variable, using an optimal temperature model of which training has been completed; acquiring predictive property values of the material from second input data, which includes at least one of the appropriate candidate temperatures calculated by the optimal temperature model of which training has been completed, the thickness of the material to be heated in the furnace, the steel grade of the material, and the operating speed of the furnace as a variable, using a property prediction model of which training has been completed; comparing the target property value with the predictive property values calculated by the property prediction model of which training has been completed and sampling the plurality of appropriate candidate temperatures to acquire one or more sampled appropriate candidate temperatures; acquiring heat information of a case of operating the furnace at the sampled appropriate candidate temperatures, comparing the heat information, and filtering the sampled appropriate candidate temperatures to acquire one or more appropriate temperatures; performing predetermined computation on the one or more appropriate temperatures to acquire an optimal temperature; and transmitting a request to set the acquired optimal temperature to a set temperature value of the furnace.

According to another aspect of the present disclosure, there is provided an electronic device including: a transceiver configured to acquire a target property value of a material input by an operator; and a processor configured to acquire a plurality of appropriate candidate temperatures of a furnace from first input data, which includes at least one of a thickness of the material to be heated in the furnace, a steel grade of the material, an operating speed of the furnace, and the target property value as a variable, using an optimal temperature model of which training has been completed, acquire predictive property values of the material from second input data, which includes at least one of the appropriate candidate temperatures calculated by the optimal temperature model of which training has been completed, the thickness of the material to be heated in the furnace, the steel grade of the material, and the operating speed of the furnace as a variable, using a property prediction model of which training has been completed, acquire one or more sampled appropriate candidate temperatures by comparing the target property value with the predictive property values calculated by the property prediction model of which training has been completed and sampling the plurality of appropriate candidate temperatures, acquire heat information of a case of operating the furnace at the sampled appropriate candidate temperatures, acquire one or more appropriate temperatures by comparing the heat information and filtering the sampled appropriate candidate temperatures, acquire an optimal temperature by performing predetermined computation on the one or more appropriate temperatures, and transmit a request to set the acquired optimal temperature to a set temperature value of the furnace.

Solutions of the present disclosure are not limited to those described above, and other solutions which have not been described will be clearly understood by those skilled in the technical field to which the present disclosure pertains from this specification and the accompanying drawings.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings.
FIG. 1 is a schematic block diagram of a system for optimizing operation of a furnace according to an exemplary embodiment of the present disclosure.
FIG. 2 is a diagram illustrating the system for optimizing operation of a furnace according to the exemplary embodiment of the present disclosure.
FIG. 3 is a diagram illustrating operations of an electronic device according to an exemplary embodiment of the present disclosure.
FIG. 4 is a flowchart illustrating a method of calculating an optimal temperature of a furnace according to an exemplary embodiment of the present disclosure.
FIG. 5 is a diagram illustrating a method of training an optimal temperature model and a phase of calculating appropriate candidate temperatures using an optimal temperature model of which training has been completed according to an exemplary embodiment of the present disclosure.
FIG. 6 is a diagram illustrating a method of training a property prediction model and a phase of calculating predictive property values using a property prediction model of which training has been completed according to an exemplary embodiment of the present disclosure.
FIG. 7 is a diagram illustrating a phase of calculating an optimal temperature from appropriate candidate temperatures according to an exemplary embodiment of the present disclosure.
FIG. 8 is a diagram illustrating a phase of a user interface.
FIG. 9 is a diagram illustrating a phase of the user interface.
FIG. 10 is a diagram illustrating a phase of the user interface.
FIG. 11 is a diagram illustrating a phase of the user interface.

### DETAILED DESCRIPTION OF EXEMPLARY EMBODIMENTS

The foregoing objects, features, and advantages of the present disclosure will become apparent through the following detailed description associated with the accompanying drawings. However, the present disclosure may be modified in various ways and have a variety of embodiments, and specific embodiments will be illustrated in the drawings and described in detail below.

Throughout the specification, like reference numerals refer to like components in principle. Also, components having the same function within the same spirit will be described using the same reference numeral, and overlapping description thereof will be omitted.

When it is determined that detailed description of a well-known function or component associated with the present disclosure may unnecessarily obscure the subject matter of the present disclosure, the detailed description will be omitted. Also, numbers (e.g., first, second, and the like) used in the process of describing this specification are merely identifiers for distinguishing one component from others.

The suffixes "module" and "part" for components used in embodiments below are assigned or mixed in consideration of only easiness in writing the specification and do not have distinctive meanings or roles.

In embodiments below, singular expressions include plural expressions unless the context clearly indicates otherwise.

In embodiments below, the terms "include," "have," and the like indicate the presence of a feature or component described in the specification and do not preclude the possibility that one or more other features or components will be added.

In the drawings, the sizes of components may be exaggerated or reduced for convenience of description. For example, the size and thickness of each component shown in the drawings are arbitrarily shown for convenience description, and the present disclosure is not necessarily limited to those illustrated.

When an embodiment can be implemented in a different manner, a specific process may be performed in a different order from that described below. For example, two processes which are consecutively described may be performed substantially at the same time or performed in an order opposite to a described order.

In embodiments below, when it is described that components and the like are connected, the components may be directly connected or indirectly connected with other components interposed therebetween.

For example, when it is described in this specification that components and the like are electrically connected, the components and the like may be directly electrically connected or indirectly electrically connected with other components and the like interposed therebetween.

A method of calculating an optimal temperature of a furnace according to an exemplary embodiment of the present disclosure may include an operation of acquiring a target property value of a material input by an operator, an operation of acquiring a plurality of appropriate candidate temperatures of the furnace from first input data, which includes at least one of a thickness of the material to be heated in the furnace, a steel grade of the material, an operating speed of the furnace, and the target property value as a variable, using an optimal temperature model of which training has been completed, an operation of acquiring predictive property values of the material from second input data, which includes at least one of the appropriate candidate temperatures calculated by the optimal temperature model of which training has been completed, the thickness of the material to be heated in the furnace, the steel grade of the material, and the operating speed of the furnace as a variable, using a property prediction model of which training has been completed, an operation of comparing the target property value with the predictive property values calculated by the property prediction model of which training has been completed and sampling the plurality of appropriate candidate temperatures to acquire one or more sampled appropriate candidate temperatures, an operation of acquiring heat information of a case of operating the furnace at the sampled appropriate candidate temperatures, comparing the heat information, and filtering the sampled appropriate candidate temperatures to acquire one or more appropriate temperatures, an operation of calculating an optimal temperature on the basis of the one or more appropriate temperatures, and an operation of transmitting a request to set the calculated optimal temperature to a set temperature value of the furnace.

According to an exemplary embodiment of the present disclosure, the operation of sampling the plurality of appropriate candidate temperatures to acquire the one or more sampled appropriate candidate temperatures may further include an operation of comparing the target property value with the predictive property values to sort the plurality of appropriate candidate temperatures in increasing order of differences between the target property value and the predictive property values and an operation of determining the appropriate candidate temperatures ranked a predetermined ordinal number or higher as the sampled appropriate candidate temperatures on the basis of a result of sorting.

According to an exemplary embodiment of the present disclosure, the operation of sampling the plurality of appropriate candidate temperatures to acquire the one or more sampled appropriate candidate temperatures may further include an operation of calculating differences between the target property value and the predictive property values by comparing the target property value with the predictive property values and an operation of determining the appropriate candidate temperatures of which the calculated differences are a predetermined value or less as the sampled appropriate candidate temperatures.

According to an exemplary embodiment of the present disclosure, the operation of filtering the sampled appropriate candidate temperatures to acquire the one or more appropriate temperatures may further include an operation of sorting the sampled appropriate candidate temperatures in decreasing order of heat on the basis of the heat information and an operation of determining the sampled appropriate candidate temperatures ranked a predetermined ordinal number or higher as the one or more appropriate temperatures on the basis of a result of sorting.

According to an exemplary embodiment of the present disclosure, the calculating of the optimal temperature on the basis of the one or more appropriate temperatures may further include an operation of calculating an average of the appropriate temperatures as the optimal temperature.

According to an exemplary embodiment of the present disclosure, the optimal temperature model may be trained on the basis of a training dataset including first training data about at least one of the thickness of the material, the steel grade of the material, the operating speed of the furnace, and the target property value, and an operating temperature of the furnace corresponding to the first training data, and specifically, the optimal temperature model may be trained on the basis of the first training data by updating parameters of the optimal temperature model to output a value close to the operating temperature of the furnace.

According to an exemplary embodiment of the present disclosure, the property prediction model may be trained on the basis of a training dataset including second training data about at least one of the thickness of the material, the steel grade of the material, the operating speed of the furnace, and an operating temperature of the furnace and property information of a case where the material is heat-treated under an operational condition corresponding to the second training data, and specifically, the property prediction model may be trained on the basis of the second training data by updating parameters of the property prediction model to output a value close to the property information.

According to an exemplary embodiment of the present disclosure, the target property value and the predictive property values may be related to at least one of tensile strength, yield strength, hardness, and elongation of the material heat-treated through the furnace.

An exemplary embodiment of the present disclosure may provide a computer-readable recording medium on which a program for performing the method of calculating an optimal temperature of a furnace is recorded.

An electronic device according to an exemplary embodiment of the present disclosure may include a transceiver configured to acquire a target property value of a material input by an operator and a processor configured to acquire a plurality of appropriate candidate temperatures of a furnace from first input data, which includes at least one of a thickness of the material to be heated in the furnace, a steel grade of the material, an operating speed of the furnace, and the target property value as a variable, using an optimal temperature model of which training has been completed, acquire predictive property values of the material from second input data, which includes at least one of the appropriate candidate temperatures calculated by the optimal temperature model of which training has been completed, the thickness of the material to be heated in the furnace, the steel grade of the material, and the operating speed of the furnace as a variable, using a property prediction model of which training has been completed, acquire one or more sampled appropriate candidate temperatures by comparing the target property value with the predictive property values calculated by the property prediction model of which training has been completed and sampling the plurality of appropriate candidate temperatures, acquire heat information of a case of operating the furnace at the sampled appropriate candidate temperatures, acquire one or more appropriate temperatures by comparing the heat information and filtering the sampled appropriate candidate temperatures, calculate an optimal temperature on the basis of the one or more appropriate temperatures, and transmit a request to set the calculated optimal temperature to a set temperature value of the furnace.

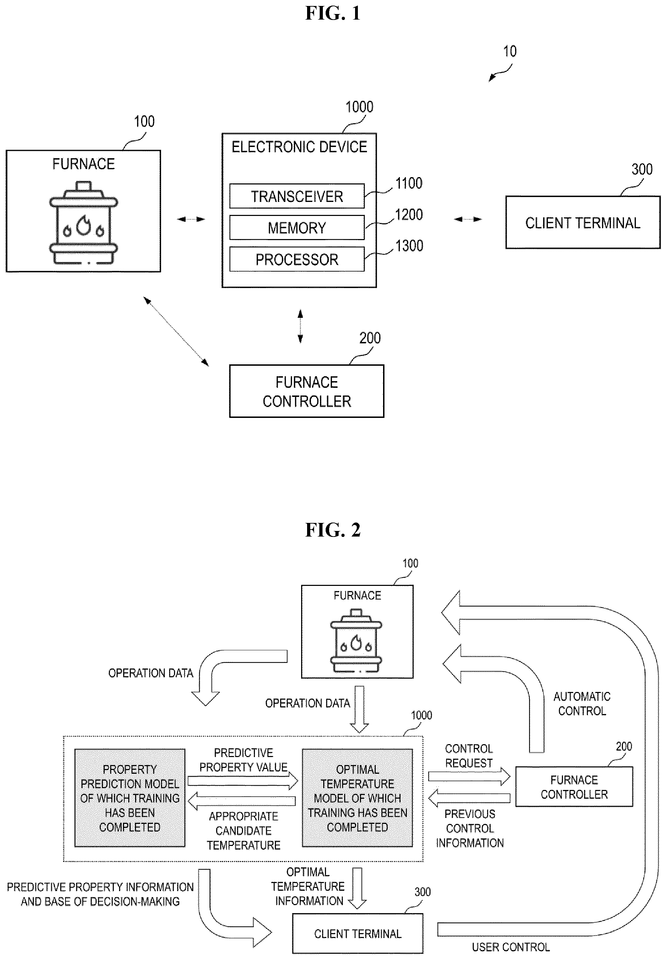

Hereinafter, a method of calculating an optimal temperature of a furnace, an electronic device (or server, hereinafter "electronic device") performing the method, and/or a system for optimizing operation of a furnace according to exemplary embodiments of the present disclosure will be described with reference to FIGS. 1 to 7.

FIG. 1 is a schematic block diagram of a system 10 for optimizing operation of a furnace according to an exemplary embodiment of the present disclosure. FIG. 2 is a diagram illustrating the system 10 for optimizing operation of a furnace according to the exemplary embodiment of the present disclosure.

The system 10 for optimizing operation of a furnace according to the exemplary embodiment of the present disclosure may include a furnace 100, a furnace controller 200, a client terminal 300, and/or an electronic device 1000.

The furnace 100 according to the exemplary embodiment of the present disclosure may transmit operation data to any external device or any external server including a furnace operation database and/or the electronic device 1000 through any transceiver. Here, the operation data may be any data related to operation of the furnace including operational conditions, such as a thickness of a material, a steel grade of the material, an operating speed of the furnace, and an operating temperature of the furnace, and a property value of a product produced under the operational conditions. Also, the furnace 100 may set an optimal temperature calculated by the electronic device 1000 to a set temperature value to heat-process a material (e.g., a steel material).

The electronic device 1000 according to the exemplary embodiment of the present disclosure may perform an operation of calculating appropriate candidate temperatures using an optimal temperature model of which training has been completed. Also, the electronic device 1000 may perform an operation of calculating a predictive property value of a product when the furnace is operated on the basis of an appropriate candidate temperature which is calculated using a property prediction model of which training has been completed. Further, the electronic device 1000 may perform an operation of calculating an optimal temperature on the basis of the calculated predictive property value and an aimed target property value. Here, the electronic device 1000 may perform an operation of transmitting a request to control the operating temperature of the furnace 100 on the basis of the calculated optimal temperature to the furnace controller 200 through a transceiver 1100. Alternatively, the electronic device 1000 may perform an operation of transmitting the calculated optimal temperature information, the predictive property information, and a base of decision-making to the client terminal 300 through the transceiver 1100.

The furnace controller 200 according to the exemplary embodiment of the present disclosure may receive the request to control the operating temperature of the furnace 100 from the electronic device 1000 through any transceiver. Here, the furnace controller 200 may perform an operation of setting the calculated optimal temperature as the set temperature value of the furnace 100 and automatically controlling the operating temperature of the furnace 100 to the set temperature value on the basis of the operating temperature and the set temperature value of the furnace 100. Meanwhile, it is shown in FIGS. 1 and 2 that the furnace controller 200 is a separate device from the furnace 100, the client terminal 300, and the electronic device 1000. However, this is for convenience of description, and the furnace controller 200 may be integrated with the furnace 100, the client terminal 300, and/or the electronic device 1000. Also, the furnace controller 200 may be provided in the form of a program that is executable on the furnace 100, the client terminal 300, and/or the electronic device 1000.

The client terminal 300 according to the exemplary embodiment of the present disclosure may receive the optimal temperature information calculated by the electronic device 1000 through any transceiver. Here, the client terminal 300 may receive a user's request to set the calculated optimal temperature information to the set temperature value of the furnace 100 through any input part (e.g., a mouse, a keyboard, a touchpad, or the like). Also, the client terminal 300 may transmit a request to control (e.g., user control of FIG. 2) the operating temperature of the furnace 100 to the set temperature value which is set as the optimal temperature on the basis of the user's request. Meanwhile, the client terminal 300 may receive the calculated optimal temperature information, the predictive property information, and/or the base of decision-making through any transceiver and provide the received information to the user through a user interface.

Referring back to FIG. 1, the electronic device 1000 according to the exemplary embodiment of the present disclosure may include the transceiver 1100, a memory 1200, and a processor 1300.

The transceiver 1100 of the electronic device 1000 may communicate with any external device or external server. For example, the electronic device 1000 may acquire operation data from the furnace 100 through the transceiver 1100. Here, the operation data may be any data related to operation of the furnace including operational conditions of the furnace, such as a thickness of a material, a steel grade of the material, an operating speed of the furnace, and an operating temperature of the furnace, and a property value of a product produced under the operational conditions. For example, the electronic device 1000 may receive, through the transceiver 1100, inference data which includes structural information, hierarchical information, computational information, and/or parameter information of the optimal temperature model of which training has been completed to properly execute the optimal temperature model of which training has been completed. For example, the electronic device 1000 may receive, through the transceiver 1100, inference data which includes structural information, hierarchical information, computational information, and/or parameter information of the property prediction model of which training has been completed to properly execute the property prediction model of which training has been completed. For example, the electronic device 1000 may transmit any analysis information including the calculated optimal temperature information, the predictive property information, and/or the base of decision-making to any external device or any external server including the client terminal 300 through the transceiver 1100. For example, the electronic device 1000 may transmit the request to set the calculated optimal temperature to the set temperature value to any external device or any external server including the furnace controller 200 through the transceiver 1100.

The electronic device 1000 may access a network through the transceiver 1100 to transmit and receive various data. The transceiver 1100 may be of a wired type or a wireless type. Since the wired type and wireless type have their own advantages and disadvantages, the electronic device 1000 of both wired and wireless types may be provided in the electronic device 1000 in some cases. The wireless-type electronic device 1000 may employ a wireless local area network (WLAN) communication method such as Wi-Fi. Alternatively, the wireless-type electronic device 1000 may employ a cellular communication method such as Long Term Evolution (LTE) or Fifth Generation (5G). However, wireless communication protocols are not limited to those mentioned above, and any appropriate wireless communication method may also be used. The wired-type electronic device 1000 may generally employ local area network (LAN) or Universal Serial Bus (USB) communication, and other methods may also be used.

The memory 1200 of the electronic device 1000 may store various information. In the memory 1200, various data may be temporarily or semi-temporarily stored. Examples of the memory 1200 may be a hard disk drive (HDD), a solid state drive (SSD), a flash memory, a read-only memory (ROM), a random access memory (RAM), and the like. The memory 1200 may be provided in a form in which it is installed in the electronic device 1000 or detachable from the electronic device 1000. In the memory 1200, various data required for operating the electronic device 1000, such as an operating system (OS) for operating the electronic device 1000 and a program for operating each element of the electronic device 1000, may be stored.

The processor 1300 may control overall operations of the electronic device 1000. For example, the processor 1300 may perform the overall operations of the electronic device 1000 including an operation of acquiring a target property value which will be described below, an operation of acquiring a plurality of appropriate candidate temperatures of the furnace using the optimal temperature model of which training has been completed, an operation of acquiring predictive property values of the material using the property prediction model of which training has been completed, an operation of acquiring sampled appropriate candidate temperatures by sampling the plurality of appropriate candidate temperatures, an operation of acquiring appropriate temperatures on the basis of heat information of a case of operating the furnace at the sampled appropriate candidate temperatures, an operation of calculating an optimal temperature on the basis of the appropriate temperatures, and/or other operations. Specifically, the processor 1300 may load a program for the overall operations of the electronic device 1000 from the memory 1200 and execute the program. The processor 1300 may be implemented in the form of an application processor (AP), a central processing unit (CPU), a microcontroller unit (MCU), or a similar device thereto as hardware, software, or a combination thereof. As hardware, the processor 1300 may be provided in the form of an electronic circuit for processing an electrical signal to perform a control function. As software, the processor 1300 may be provided in the form of a program or code for operating a hardware circuit.

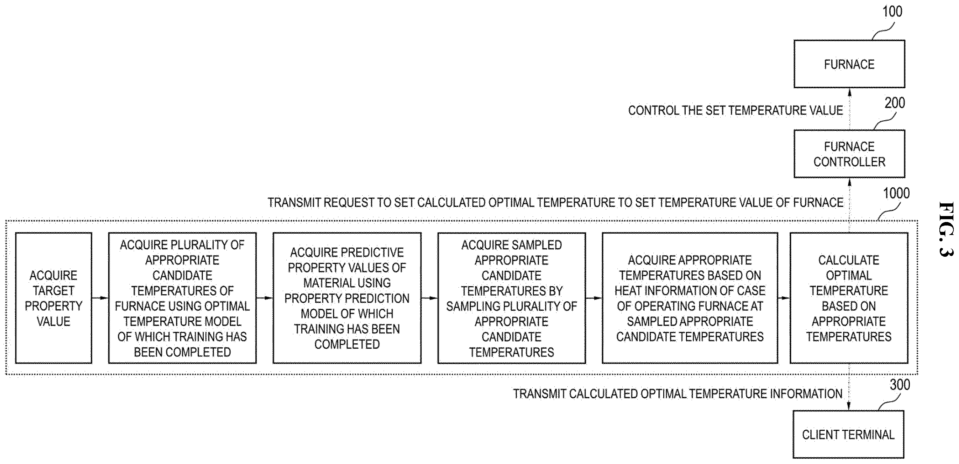

FIG. 3 is a diagram illustrating operations of the electronic device 1000 according to an exemplary embodiment of the present disclosure.

The electronic device 1000 according to the exemplary embodiment of the present disclosure may acquire a target property value through the transceiver 1100. Specifically, through the transceiver 1100, the electronic device 1000 may acquire a target property value which is input by an operator of the furnace 100 regarding aimed property information of a manufactured product. Here, the target property value may be any aimed property value of a heat-treated material (or a heat-treated product) including tensile strength, yield strength, hardness, and elongation of the material heat-treated through the furnace.

The electronic device 1000 according to the exemplary embodiment of the present disclosure may acquire inference data of the optimal temperature model of which training has been completed through the transceiver 1100. Here, the inference data of the optimal temperature model of which training has been completed may be any data which includes structural information, hierarchical information, computational information, and/or parameter information of the optimal temperature model of which training has been completed to properly execute the optimal temperature model of which training has been completed.

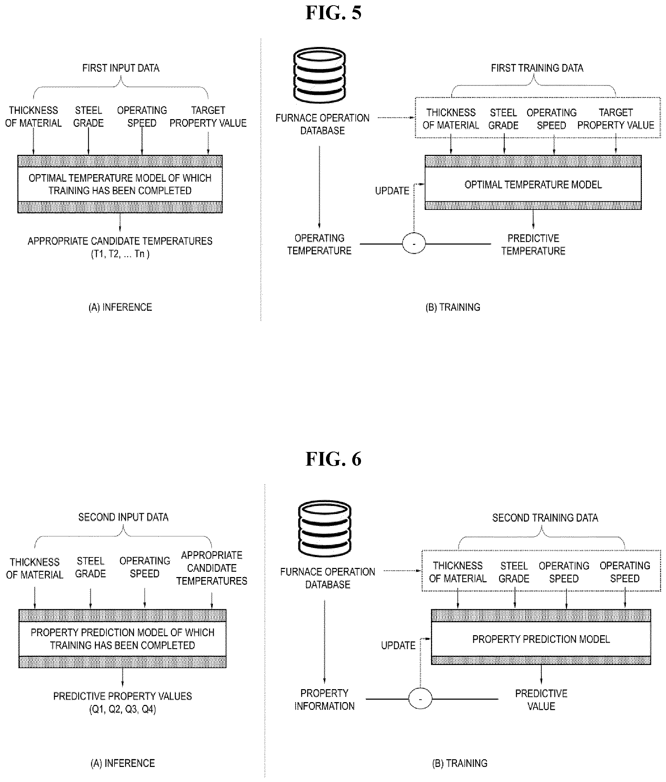

The electronic device 1000 according to the exemplary embodiment of the present disclosure may acquire a plurality of appropriate candidate temperatures of the furnace using the optimal temperature model of which training has been completed. More specifically, the electronic device 1000 may input first input data to an input layer of the optimal temperature model of which training has been completed and acquire a plurality of appropriate candidate temperatures through an output layer of the optimal temperature model of which training has been completed. Here, the first input data may include a thickness of the material to be heated in the furnace, a steel grade of the material, an operating speed of the furnace, the target property value, and/or a combination thereof as a variable. Meanwhile, the optimal temperature model may be trained on the basis of a training dataset including first training data about the thickness of the material, the steel grade of the material, the operating speed of the furnace, and/or the target property value which are obtained from the furnace operation database and an actual operating temperature of the furnace corresponding to the first training data. More specifically, the optimal temperature model may be trained on the basis of the first training data by updating parameters (weights) of the optimal temperature model to output a value close to the actual operating temperature corresponding to the first training data. Therefore, the optimal temperature model of which training has been completed may be configured to calculate an appropriate candidate temperature corresponding to the actual operating temperature of the furnace on the basis of the first input data corresponding to the variable of the first training data. A method of training the optimal temperature model and/or an inference phase of the optimal temperature model will be described in further detail with reference to FIG. 5.

The electronic device 1000 according to an exemplary embodiment of the present disclosure may acquire inference data of the property prediction model of which training has been completed through the transceiver 1100. Here, the inference data of the property prediction model of which training has been completed may be any data including structural information, hierarchical information, computational information, and/or parameter information of the property prediction model of which training has been completed to properly execute the property prediction model of which training has been completed.

The electronic device 1000 according to the exemplary embodiment of the present disclosure may acquire predictive property values of the material using the property prediction model of which training has been completed. Specifically, the electronic device 1000 may input second input data to an input layer of the property prediction model of which training has been completed and acquire predictive property values of the material through an output layer of the property prediction model of which training has been completed. Here, the second input data may include the appropriate candidate temperature calculated by the optimal temperature model of which training has been completed, the thickness of the material, the steel grade of the material, the operating speed of the furnace, and/or a combination thereof as a variable. Meanwhile, the property prediction model may be trained on the basis of a training dataset including second training data about at least one of the thickness of the material, the steel grade of the material, the operating speed of the furnace, and/or the operating temperature which are obtained from the furnace operation database and property information (e.g., tensile strength, yield strength, hardness, and/or elongation of the material) of a case where the material is heat-treated under an operational condition corresponding to the second training data. More specifically, the property prediction model may be trained on the basis of the second training data by updating parameters (weights) of the property prediction model to output a value close to property information of a case where the material is heat-treated under an operational condition corresponding to the second training data. Therefore, the property prediction model of which training has been completed may be configured to calculate, on the basis of the second input data corresponding to the variable of the second training data, a predictive property value corresponding to property information of a case where the material is heat-treated under an operational condition corresponding to the second input data. A method of training the property prediction model and/or an inference phase of the property prediction model will be described in further detail with reference to FIG. 6.

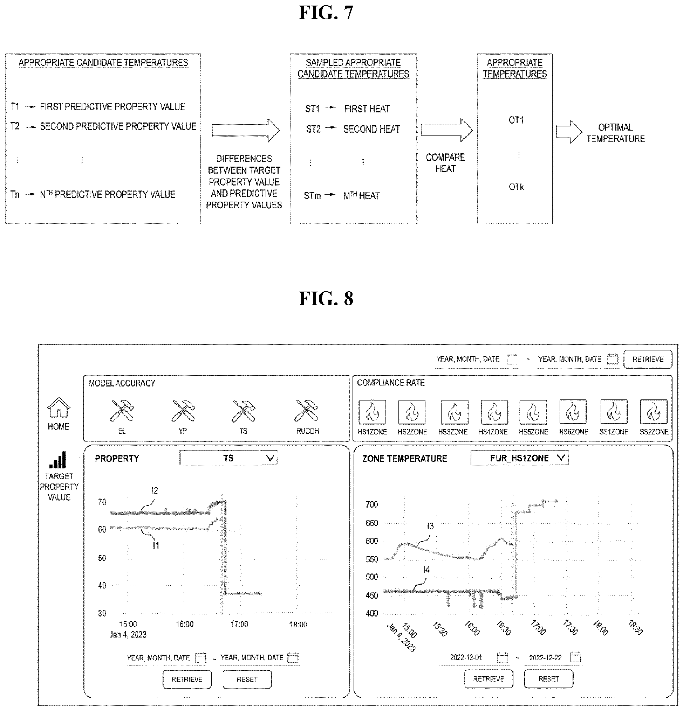

The electronic device 1000 according to the exemplary embodiment of the present disclosure may perform an operation of acquiring sampled appropriate candidate temperatures by sampling a plurality of appropriate candidate temperatures. Specifically, the electronic device 1000 may compare the target property value which is input by the operator with the predictive property values calculated by the property prediction model of which training has been completed and acquire one or more sampled appropriate candidate temperatures by sampling the plurality of appropriate candidate temperatures calculated by the optimal temperature model of which training has been completed. For example, the electronic device 1000 may compare the target property value with the predictive property values to sort the plurality of appropriate candidate temperatures in increasing order of difference between the target property value and the predictive property values. Here, the electronic device 1000 may determine the appropriate candidate temperatures corresponding to predictive property values ranked a predetermined ordinal number or higher as sampled appropriate candidate temperatures on the basis of a result of sorting. For example, the electronic device 1000 may calculate a difference between the target property value and a predictive property value by comparing the target property value with the predictive property value. Here, the electronic device 1000 may determine appropriate candidate temperatures corresponding to predictive property values of which calculated differences are a predetermined value or less as sampled appropriate candidate temperatures. Sampling a plurality of appropriate candidate temperatures will be described in further detail with reference to FIG. 7.

The electronic device 1000 according to the exemplary embodiment of the present disclosure may perform an operation of acquiring appropriate temperatures on the basis of heat information of a case of operating the furnace at the sampled appropriate candidate temperatures. Specifically, the electronic device 1000 may acquire heat information of a case of operating the furnace at each of the sampled appropriate candidate temperatures, compare the heat information, and filter the sampled appropriate candidate temperatures to acquire one or more appropriate temperatures. For example, the electronic device 1000 may sort the sampled appropriate candidate temperatures in increasing order of heat on the basis of the heat information. Here, the electronic device 1000 may determine sampled appropriate candidate temperatures corresponding to heat of a predetermined ranking as one or more appropriate temperatures on the basis of a result of sorting. Meanwhile, the heat information may be calculated on the basis of the sum of appropriate candidate temperatures for zones of the furnace or the amount of fuel (e.g., liquefied natural gas (LNG)) or electrical energy required for zone-specific appropriate candidate temperatures. Filtering sampled appropriate candidate temperatures will be described in further detail with reference to FIG. 7.

The electronic device 1000 according to the exemplary embodiment of the present disclosure may perform an operation of calculating an optimal temperature on the basis of the appropriate temperatures. For example, the electronic device 1000 may calculate an average of the appropriate temperatures as an optimal temperature. Calculating an optimal temperature will be described in further detail with reference to FIG. 7.

The electronic device 1000 according to the exemplary embodiment of the present disclosure may transmit a request to set the calculated optimal temperature to a set temperature value of the furnace to the furnace controller 200 through the transceiver 1100. Here, the furnace controller 200 may set the calculated optimal temperature to the set temperature value of the furnace 100 in response to the request. Also, the furnace controller 200 may perform an operation of automatically controlling the operating temperature of the furnace to the set temperature value on the basis of the operating temperature and the set temperature value of the furnace 100.

Meanwhile, the electronic device 1000 according to the exemplary embodiment of the present disclosure may transmit the calculated optimal temperature information to the client terminal 300 through the transceiver 1100. Here, the client terminal 300 may receive an input to control the operating temperature of the furnace using the calculated optimal temperature information. Also, the client terminal 300 may transmit a request to set the calculated optimal temperature to the set temperature value of the furnace 100 to the furnace 100 in response to the received input.

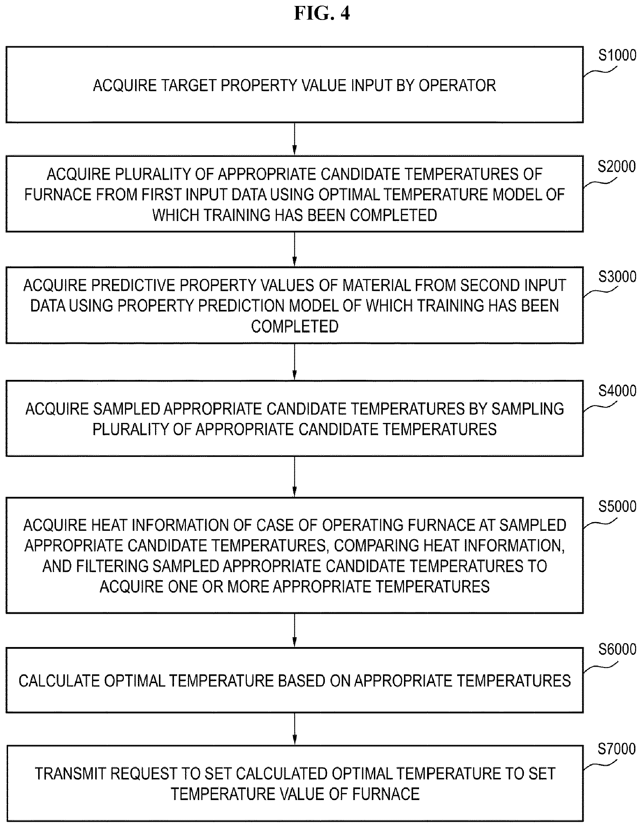

Operations of the electronic device 1000 and a method of calculating an optimal temperature of a furnace which is performed by the electronic device 1000 according to an exemplary embodiment of the present disclosure will be described in detail below with reference to FIGS. 4 to 7. In describing the method of calculating an optimal temperature of a furnace, some embodiments that overlap the above description of FIGS. 1 to 3 may be omitted. However, this is only for convenience of description, and the present disclosure is not limited thereto.

FIG. 4 is a flowchart illustrating a method of calculating an optimal temperature of a furnace according to an exemplary embodiment of the present disclosure.

The method of calculating an optimal temperature of a furnace according to the exemplary embodiment of the present disclosure may include an operation S1000 of acquiring a target property value of a material input by an operator, an operation S2000 of acquiring a plurality of appropriate candidate temperatures of the furnace from first input data using an optimal temperature model of which training has been completed, an operation S3000 of acquiring predictive property values of the material from second input data using a property prediction model of which training has been completed, an operation S4000 of sampling the plurality of appropriate candidate temperatures to acquire one or more sampled appropriate candidate temperatures, an operation S5000 of acquiring heat information of a case of operating the furnace at the sampled appropriate candidate temperatures, comparing the heat information, and filtering the sampled appropriate candidate temperatures to acquire one or more appropriate temperatures, an operation S6000 of calculating an optimal temperature on the basis of the one or more appropriate temperatures, and an operation S7000 of transmitting a request to set the acquired optimal temperature to a set temperature value of the furnace.

In the operation S1000 of acquiring the target property value of the material input by the operator, the electronic device 1000 may acquire the target property value of aimed property information of the product input by the operator of the furnace. Here, the target property value may be an aimed value of any property of the material heat-treated in the furnace such as tensile strength, yield strength, hardness, and/or elongation of the heat-treated material.

In the operation S2000 of acquiring the plurality of appropriate candidate temperatures of the furnace from the first input data using the optimal temperature model of which training has been completed, the electronic device 1000 may acquire the plurality of appropriate candidate temperatures of the furnace from the first input data which includes a thickness of the material to be heated in the furnace, a steel grade of the material, an operating speed of the furnace, the target property value acquired through the operation S1000, and/or a combination thereof as a variable.

FIG. 5 is a diagram illustrating a method of training an optimal temperature model and a phase of calculating appropriate candidate temperatures using an optimal temperature model of which training has been completed according to an exemplary embodiment of the present disclosure. Specifically, FIG. 5A is a diagram showing a phase of calculating appropriate candidate temperatures using an optimal temperature model of which training has been completed according to the exemplary embodiment of the present disclosure, and FIG. 5B is a diagram showing a method of training an optimal temperature model according to the exemplary embodiment of the present disclosure.

The optimal temperature model of which training has been completed may acquire first input data through an input layer, calculate a plurality of appropriate candidate temperatures (T1, T2, ..., and Tn of FIG. 5A) according to parameters (or weights) updated through the training, and output the calculated appropriate candidate temperatures through an output layer. Here, the electronic device 1000 may acquire the plurality of appropriate candidate temperatures through the output layer of the optimal temperature model of which training has been completed.

Meanwhile, the optimal temperature model may be trained on the basis of a training dataset including first training data including a thickness of a material, a steel grade of the material, an operating speed of a furnace, a target property value, and/or a combination thereof which are obtained from the furnace operation database and an operating temperature of the furnace corresponding to the first training data. More specifically, the optimal temperature model may output a predictive temperature on the basis of the first training data. Here, the optimal temperature model may be trained on the basis of a difference between the predictive temperature and the operating temperature of the furnace included in the training dataset by updating parameters (or weights) of the optimal temperature model to output a value close to the operating temperature of the furnace as the predictive temperature. Accordingly, the optimal temperature model of which training has been completed may calculate appropriate candidate temperatures corresponding to an actual operating temperature of the furnace on the basis of the first input data corresponding to a variable of the first training data.

In the operation S3000 of acquiring the predictive property values of the material from the second input data using the property prediction model of which training has been completed, the electronic device 1000 may acquire predictive property values of the material from the second input data, which includes the appropriate candidate temperatures calculated through the operation S2000, the thickness of the material, the steel grade of the material, and the operating speed of the furnace, and/or a combination thereof as a variable, using the property prediction model of which training has been completed. Here, the predictive property values may be predictive values of any property of the material to be heat treated in the furnace such as tensile strength, yield strength, hardness, and/or elongation of the heat-treated material.

FIG. 6 is a diagram illustrating a method of training a property prediction model and a phase of calculating predictive property values using a property prediction model of which training has been completed according to an exemplary embodiment of the present disclosure. Specifically, FIG. 6A is a diagram showing a phase of calculating a predictive property value using a property prediction model of which training has been completed according to the exemplary embodiment of the present disclosure, and FIG. 6B is a diagram showing a method of training a property prediction model according to the exemplary embodiment of the present disclosure.

The property prediction model of which training has been completed may acquire second input data through an input layer, calculate predictive property values (e.g., a predictive property value Q1 related to tensile strength, a predictive property value Q2 related to yield strength, a predictive property value Q3 related to hardness, and a predictive property value Q4 related to elongation of FIG. 6A) according to parameters (or weights) updated through the training, and output the calculated predictive property values through an output layer. Here, the electronic device 1000 may acquire the predictive property values through the output layer of the property prediction model of which training has been completed.

Meanwhile, the property prediction model may be trained on the basis of a training dataset including second training data including a thickness of a material, a steel grade of the material, an operating speed of a furnace, an operating temperature of the furnace, and/or a combination thereof which are obtained from the furnace operation database and property information of a case where the material is heat-treated under an operational condition corresponding to the second training data. More specifically, the property prediction model may output a predictive value related to property information on the basis of the second training data. Here, the property prediction model may be trained on the basis of a difference between the predictive value and the property information included in the training dataset by updating parameters (or weights) of the property prediction model to output a value close to the property information as the predictive value. Accordingly, the property prediction model of which training has been completed may calculate, for example, a predictive property value of a case of heat-processing the material at an appropriate candidate temperature calculated by an optimal temperature model of which training has been completed on the basis of the second input data corresponding to a variable of the second training data under the operational condition corresponding to the second training data.

FIG. 7 is a diagram illustrating a phase of calculating an optimal temperature from appropriate candidate temperatures according to an exemplary embodiment of the present disclosure.

In the operation S4000 of sampling the plurality of appropriate candidate temperatures to acquire the one or more sampled appropriate candidate temperatures, the electronic device 1000 may perform an operation of sampling the plurality of appropriate candidate temperatures to acquire sampled appropriate candidate temperatures. Specifically, the electronic device 1000 may compare the target property value input by the operator with the predictive property values that are calculated on the basis of the appropriate candidate temperatures by the property prediction model of which training has been completed and sample the plurality of appropriate candidate temperatures calculated by the optimal temperature model of which training has been completed. For example, the electronic device 1000 may calculate a first difference value between the target property value input by the operator and a first predictive property value that is calculated on the basis of a first appropriate candidate temperature (e.g., T1 of FIG. 7) by the property prediction model of which training has been completed. For example, the electronic device 1000 may calculate a second difference value between the target property value input by the operator and a second predictive property value that is calculated on the basis of a second appropriate candidate temperature (e.g., T2 of FIG. 7) by the property prediction model of which training has been completed. For example, the electronic device 1000 may calculate an n^{th} difference value between the target property value input by the operator and an n^{th} predictive property value that is calculated on the basis of an n^{th} appropriate candidate temperature (e.g., Tn of FIG. 7) by the property prediction model of which training has been completed.

As an example, the electronic device 1000 may sort the plurality of appropriate candidate temperatures in increasing order of the difference value between the target property value and a predictive property value. Also, the electronic device 1000 may determine the appropriate candidate temperatures corresponding to predictive property values ranked a predetermined ordinal number or higher as sampled appropriate candidate temperatures (ST1, ST2, ..., and STm of FIG. 7) on the basis of a result of sorting. For example, the electronic device 1000 may determine appropriate candidate temperatures corresponding to predictive property values of which calculated differences are a predetermined value or less as sampled appropriate candidate temperatures (ST1, ST2, ..., and STm of FIG. 7).

However, the above description is merely illustrative, and the electronic device 1000 may sample appropriate candidate temperatures on the basis of any appropriate method.

In the operation S5000 of acquiring the heat information of the case of operating the furnace at the sampled appropriate candidate temperatures, comparing the heat information, and filtering the sampled appropriate candidate temperatures to acquire the one or more appropriate temperatures, the electronic device 1000 may acquire heat information of a case of operating the furnace at each of the sampled appropriate candidate temperatures. For example, the electronic device 1000 may acquire heat information (e.g., a first heat of FIG. 7) used in a case of operating the furnace at the sampled first appropriate candidate temperature (e.g., ST1 of FIG. 7). For example, the electronic device 1000 may acquire heat information (e.g., a second heat of FIG. 7) used in a case of operating the furnace at the sampled second appropriate candidate temperature (e.g., ST2 of FIG. 7). For example, the electronic device 1000 may acquire heat information (e.g., m^{th} heat of FIG. 7) used in a case of operating the furnace at the sampled m^{th} appropriate candidate temperature (e.g., STm of FIG. 7).

Here, the electronic device 1000 may compare the acquired heat information and filter the sampled appropriate candidate temperatures to acquire one or more appropriate temperatures. Specifically, the electronic device 1000 may sort the sampled appropriate candidate temperatures in increasing order of heat on the basis of the heat information. Here, the electronic device 1000 may determine the sampled appropriate candidate temperatures corresponding to heat ranked a predetermined ordinal number or higher on the basis of a result of sorting one or more appropriate temperatures (OT1 to OTk of FIG. 7).

However, the above description is merely illustrative, and the electronic device 1000 may acquire appropriate temperatures from sampled appropriate candidate temperatures on the basis of any appropriate method.

In the operation S6000 of calculating an optimal temperature on the basis of the one or more appropriate temperatures, the electronic device 1000 may calculate an average of the appropriate temperatures as an optimal temperature. For example, the electronic device 1000 may calculate an average of the appropriate temperatures (e.g., the first appropriate temperature OT1 to the k^{th} appropriate temperature OTk of FIG. 7) acquired through the operation S5000 and determine an average of the calculated appropriate temperatures as an optimal temperature.

Although not shown in FIG. 4, the operations S2000 to S6000 may be repeated a few times to calculate an optimal temperature in the method of calculating an optimal temperature of a furnace according to the exemplary embodiment of the present disclosure.

In the operation S7000 of transmitting the request to set the acquired optimal temperature to the set temperature value of the furnace, the electronic device 1000 may transmit a request to set the calculated optimal temperature to the set temperature value of the furnace to the furnace controller 200 through the transceiver 1100. Here, the furnace controller 200 may set the set temperature value of the furnace 100 as the calculated optimal temperature in response to the request. Also, the furnace controller 200 may perform an operation of automatically controlling the operating temperature of the furnace 100 using the set temperature value on the basis of the operating temperature and the set temperature value of the furnace 100.

A user interface that may be provided to the client terminal 300 according to an exemplary embodiment of the present disclosure will be described below with reference to FIGS. 8 to 11. FIGS. 8 to 11 are diagrams illustrating phases of a user interface.

Referring to FIG. 8, a user interface according to an exemplary embodiment of the present disclosure may output first predictive property information (I1 of FIG. 8) of a case of operating the furnace under a current operational condition and/or second predictive property information (I2 of FIG. 8) of a case of operating the furnace at an optimal temperature to a user through any output part (e.g., a display or the like) of the client terminal 300. Specifically, the electronic device 1000 may acquire the first predictive property information I1 from the current operational condition about a thickness of a material, a steel grade, a current operating speed, and/or a current operating temperature using the property prediction model of which training has been completed. Also, the electronic device 1000 may acquire the second predictive property information I2 from the current operational condition about the thickness of the material, the steel grade, the current operating speed, and/or an optimal temperature using the property prediction model of which training has been completed. Here, the electronic device 1000 may provide the first predictive property information I1 and the second predictive property information I2 to the user through the user interface.

Also, the user interface according to the exemplary embodiment of the present disclosure may output real-time temperature information (I3 of FIG. 8) and optimal temperature information (I4 of FIG. 8) of the furnace to the user through any output part (e.g., the display or the like) of the client terminal 300. Specifically, the electronic device 1000 may acquire the real-time temperature information of the furnace 100 from operation data of the furnace 100. Also, as described above, the electronic device 1000 may calculate an optimal temperature of the furnace 100 using the optimal temperature model of which training has been completed. Here, the electronic device 1000 may provide the real-time temperature information I3 and the optimal temperature information I4 of the furnace 100 to the user through the user interface.

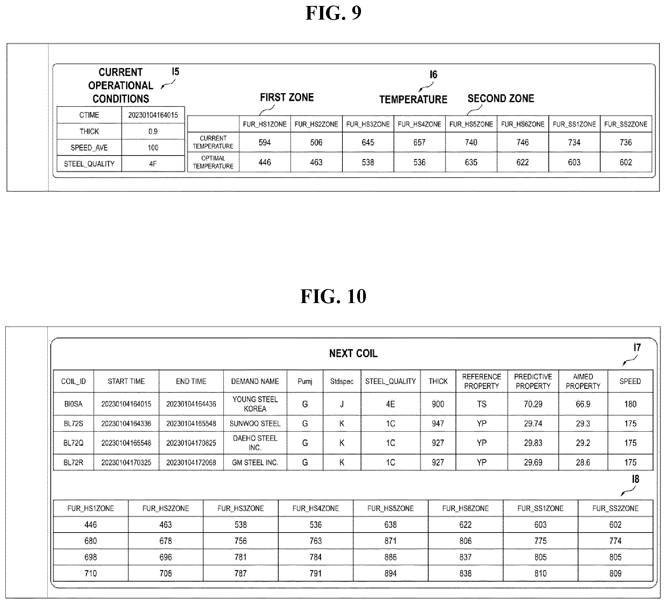

Referring to FIG. 9, the user interface according to the exemplary embodiment of the present disclosure may output a current operational condition (I5 of FIG. 9) related to a time, the thickness of the material, the operating speed, and/or a target property of the material and comparative information (I6 of FIG. 9) between the current operating temperature and an optimal temperature (or an appropriate candidate temperature or the like) calculated under the current operational condition by the optimal temperature model of which training has been completed to the user through any output part (e.g., the display or the like) of the client terminal 300. Meanwhile, the furnace 100 may include a plurality of zones. The electronic device 1000 may calculate an optimal temperature for each zone using the above-described method. Here, the electronic device 1000 may provide comparative information I6 between a current operating temperature of a first zone and an optimal temperature of the first zone and/or between a current operating temperature of a second zone and an optimal temperature of the second zone to the user.

Referring to FIG. 10, the user interface according to the exemplary embodiment of the present disclosure may output operational conditions (I7 of FIG. 10) for each operational schedule segment of the furnace to be implemented and optimal temperatures (I8 of FIG. 10) calculated from operational conditions for each operational schedule segment using the optimal temperature model of which training has been completed to the user through any output part (e.g., the display or the like) of the client terminal 300. The operational conditions I7 for each operational schedule segment may include an identifier (ID) of the material (coil), an operation start time, an operation end time, operation entity information, a thickness of the material, a predictive property of the material, an aimed property of the material, and/or an operating speed. Also, the electronic device 1000 may calculate an optimal temperature of each zone in the furnace on the basis of operational conditions for each operational schedule segment using the optimal temperature model of which training has been completed. Here, the electronic device 1000 may provide the optimal temperature I8 for each operational schedule segment of the furnace to be implemented later to the user through the user interface.

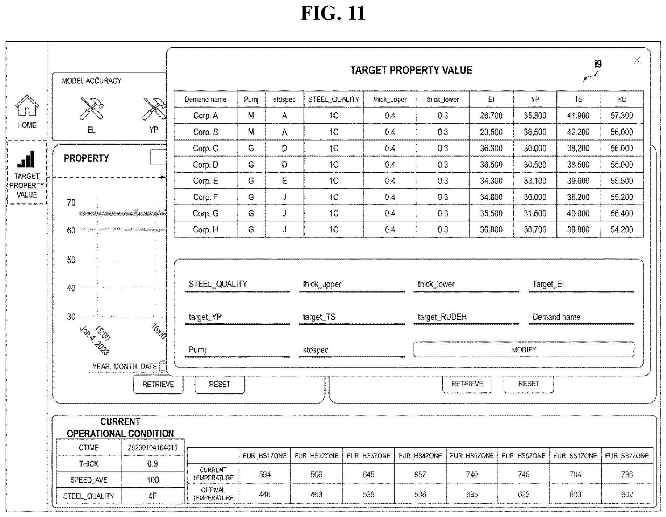

Referring to FIG. 11, the user interface according to the exemplary embodiment of the present disclosure may be provided to acquire an input of a target property value (19 of FIG. 11) through any input part (e.g., a mouse, a touchpad, a keyboard, or the like) of the client terminal 300. Specifically, the user interface may be provided to acquire an input of a target property value (e.g., steel quality and a thickness of the material (e.g., an upper thickness, a lower thickness, EI, YP, TS, HD, stdspec, pumj, and the like)). Here, the electronic device 1000 may calculate appropriate candidate temperatures using the target property value or calculate a predictive property value.

Meanwhile, the user interface shown in FIGS. 8 to 11 is merely illustrative, and the system 10 for optimizing operation of a furnace according to the exemplary embodiment of the present disclosure may acquire any appropriate information from the user or provide any appropriate information to the user through any appropriate form of user interface. Although not shown in the drawings, according to an exemplary embodiment of the present disclosure, comparative information between a predictive property based on a current operating temperature and a predictive property based on an optimal temperature may be provided to the user through the user interface as a base of calculated optimal temperature.

Various operations of the electronic device 1000 described above may be stored in the memory 1200 of the electronic device 1000, and the processor 1300 may perform the operations stored in the memory 1200.

With a method of calculating an optimal temperature of a furnace and an electronic device for performing the method according to exemplary embodiments of the present disclosure, it is possible to not only achieve an aimed property value but also calculate an optimal operating temperature of a furnace using an optimal temperature model and a property prediction model.

With a method of calculating an optimal temperature of a furnace and an electronic device for performing the method according to exemplary embodiments of the present disclosure, an optimal temperature is calculated using heat information of a case of operating a furnace at an appropriate candidate temperature sampled by an optimal temperature model and a property prediction model. Accordingly, it is possible to increase thermal efficiency of a furnace while achieving an aimed property value of a product.

With a method of calculating an optimal temperature of a furnace and an electronic device for performing the method according to exemplary embodiments of the present disclosure, an artificial intelligence (AI) model automatically calculates an optimal temperature of a furnace in consideration of an aimed property value of a product. Accordingly, the operating temperature of the furnace can be controlled to stably produce products having the aimed property value.

Effects of the present disclosure are not limited to those described above, and other effects which have not been described above will be clearly understood by those skilled in the technical field to which the present disclosure pertains from this specification and the accompanying drawings.

The features, structures, effects, and the like described in the above embodiments are included in at least one embodiment of the present disclosure and are not necessarily limited to only one embodiment. Further, features, structures, effects, and the like illustrated in each embodiment can also be implemented in combination or in a modified form according to other embodiments by those skilled in the field to which the embodiments pertain. Therefore, content related to such a combination and modification should be construed as falling within the scope of the present disclosure.
Although embodiments of the present disclosure have mainly been described, these are mere examples and do not limit the present disclosure. Those skilled in the field to which the present disclosure pertains should know that various modifications and applications not illustrated above are possible without departing from the essential characteristics of the present embodiments. In other words, each component specified in an embodiment may be implemented in a modified form. In addition, differences related to such modifications and applications should be construed as falling within the scope of the present disclosure.

## Claims

1. A method of calculating an optimal temperature of a furnace by an electronic device, the method comprising:
acquiring a target property value of a material input by an operator;
acquiring a plurality of appropriate candidate temperatures of the furnace from first input data, which includes at least one of a thickness of the material to be heated in the furnace, a steel grade of the material, an operating speed of the furnace, and the target property value as a variable, using an optimal temperature model of which training has been completed;
acquiring predictive property values of the material from second input data, which includes at least one of the appropriate candidate temperatures calculated by the optimal temperature model of which training has been completed, the thickness of the material to be heated in the furnace, the steel grade of the material, and the operating speed of the furnace as a variable, using a property prediction model of which training has been completed;
comparing the target property value with the predictive property values calculated by the property prediction model of which training has been completed and sampling the plurality of appropriate candidate temperatures to acquire one or more sampled appropriate candidate temperatures;
acquiring heat information of a case of operating the furnace at the sampled appropriate candidate temperatures, comparing the heat information, and filtering the sampled appropriate candidate temperatures to acquire one or more appropriate temperatures;
performing predetermined computation on the one or more appropriate temperatures to acquire an optimal temperature; and
transmitting a request to set the acquired optimal temperature to a set temperature value of the furnace.

2. The method of claim 1, wherein the sampling of the plurality of appropriate candidate temperatures to acquire the one or more sampled appropriate candidate temperatures further comprises:
comparing the target property value with the predictive property values to sort the plurality of appropriate candidate temperatures in increasing order of differences between the target property value and the predictive property values; and
determining the appropriate candidate temperatures ranked a predetermined ordinal number or higher as the sampled appropriate candidate temperatures on the basis of a result of sorting.

3. The method of claim 1, wherein the sampling of the plurality of appropriate candidate temperatures to acquire the one or more sampled appropriate candidate temperatures further comprises:
calculating differences between the target property value and the predictive property values by comparing the target property value with the predictive property values; and
determining the appropriate candidate temperatures of which the calculated differences are a predetermined value or less as the sampled appropriate candidate temperatures.

4. The method of claim 1, wherein the filtering of the sampled appropriate candidate temperatures to acquire the one or more appropriate temperatures further comprises:
sorting the sampled appropriate candidate temperatures in decreasing order of heat on the basis of the heat information; and
determining the sampled appropriate candidate temperatures ranked a predetermined ordinal number or higher as the one or more appropriate temperatures on the basis of a result of sorting.

5. The method of claim 1, wherein the optimal temperature is an average of the one or more appropriate temperatures.

6. The method of claim 1, wherein the optimal temperature model is trained on the basis of a training dataset including first training data about at least one of the thickness of the material, the steel grade of the material, the operating speed of the furnace, and the target property value and an operating temperature of the furnace corresponding to the first training data,
wherein the optimal temperature model is trained on the basis of the first training data by updating parameters of the optimal temperature model to output a value close to the operating temperature of the furnace.

7. The method of claim 1, wherein the property prediction model is trained on the basis of a training dataset including second training data about at least one of the thickness of the material, the steel grade of the material, the operating speed of the furnace, and an operating temperature of the furnace and property information of a case where the material is heat-treated under an operational condition corresponding to the second training data,
wherein the property prediction model is trained on the basis of the second training data by updating parameters of the property prediction model to output a value close to the property information.

8. The method of claim 1, wherein the target property value and the predictive property values are related to at least one of tensile strength, yield strength, hardness, and elongation of the material heat-treated through the furnace.

9. A non-transitory computer-readable recording medium including instructions for a computer to perform the method of claim 1.

10. An electronic device for calculating an optimal temperature of a furnace, the electronic device comprising:
a transceiver configured to acquire a target property value of a material input by an operator; and
a processor configured to:
acquire a plurality of appropriate candidate temperatures of a furnace from first input data, which includes at least one of a thickness of the material to be heated in the furnace, a steel grade of the material, an operating speed of the furnace, and the target property value as a variable, using an optimal temperature model of which training has been completed,
acquire predictive property values of the material from second input data, which includes at least one of the appropriate candidate temperatures calculated by the optimal temperature model of which training has been completed, the thickness of the material to be heated in the furnace, the steel grade of the material, and the operating speed of the furnace as a variable, using a property prediction model of which training has been completed,
acquire one or more sampled appropriate candidate temperatures by comparing the target property value with the predictive property values calculated by the property prediction model of which training has been completed and sampling the plurality of appropriate candidate temperatures,
acquire heat information of a case of operating the furnace at the sampled appropriate candidate temperatures,
acquire one or more appropriate temperatures by comparing the heat information and filtering the sampled appropriate candidate temperatures,
acquire an optimal temperature by performing predetermined computation on the one or more appropriate temperatures, and
transmit a request to set the acquired optimal temperature to a set temperature value of the furnace.
